# EUROPEAN PATENT SPECIFICATION

(11) **EP 2 047 788 B1**
(45) Date of publication and mention of the grant of the patent: **18.06.2014**
(21) Application number: 07767069.3
(22) Date of filing: 08.06.2007
(51) Int. Cl.: A61B 1/00, A61B 1/04, G02B 23/24

(54) **ENDOSCOPE SYSTEM**
ENDOSKOPSYSTEM
SYSTÈME D'ENDOSCOPE

(30) Priority: 27.07.2006 JP 2006205143
(43) Date of publication of application: 15.04.2009
(73) Proprietor: Olympus Medical Systems Corp., Tokyo 151-0072 (JP)
(72) Inventor: URAKAWA, Tsutomu, Tokyo 151-0072 (JP)
(74) Representative: von Hellfeld, Axel
(86) International application number: PCT/JP2007/061629
(87) International publication number: WO 2008/012998

(56) References cited:
- WO-A1-2005/082230
- WO-A1-2005/102144
- JP-A- 11 032 986
- JP-A- 2005 185 541
- JP-A- 2006 136 661
- JP-A- 2006 136 662
- JP-A- 2006 136 662

## Description

### Technical Field

The present invention relates to an endoscopic system having an electronic endoscope inserted in a body cavity to perform an endoscopic inspection and a display device for displaying an endoscopic image of the interior of the body cavity taken with the electronic endoscope.

### Background Art

Medical endoscopes have been used for endoscopic inspections by being inserted into body cavities. In recent years, electronic endoscopes have been known that take endoscopic images of the interiors of body cavities using an image pickup device or the like and display the images on a monitor.

An endoscopic image taken with such an electronic endoscope is recorded as an inspection recording on a film photograph or a recording medium. When this recording is performed, there is a need to correctly record, for identifying a subject, various sorts of information including information on the patient subjected to the endoscopic inspection and the date of inspection.

For example, Japanese Patent Application Laid-Open Publication No. 7-111976 discloses an electronic endoscope apparatus capable of displaying the above-described sorts of information as character lines on a monitor screen.

The conventional electronic endoscope apparatus, however, requires inputting the above-described sorts of information by means of a keyboard connected to a processor. It is, therefore, necessary for the conventional electronic endoscope apparatus to have a processor to which an electronic endoscope is wired and a space for placement of a keyboard as well as a monitor.

Since the electronic endoscope is wired to the processor, the processor can be placed only at a position close to the electronic endoscope. Correspondingly, the keyboard connected to the processor must also be placed in the vicinity of the processor and the electronic endoscope.

Under these circumstances, there has been a problem for a user, who is a doctor, that since various devices are set in a manipulation action region close to a patient, the various devices obstruct user's manipulation, making the user feel that the manipulation action region and the in-hospital space are narrow.

Also, hygienic management on the keyboard in particular is required before and during an operation, because the above-described various sorts of information are manually inputted through the keyboard. Therefore, an infection prevention measure using an antimicrobial coating or a bacteria blocking cover on the keyboard is taken.

However, it is necessary to perform a sterilization treatment on the keyboard immediately after an operation and there is, therefore, a problem that the availability factor is reduced when endoscopic inspections are successively made. Also, it is preferable, from the viewpoint of hygiene, to avoid inputting various sorts of information by means of the keyboard during an operation for endoscopic inspection.

In view of the above-described circumstances, an object of the present invention is to provide an endoscopic system which is so compact as not to be an obstacle in a user manipulation action region, and which enables a user to easily input various sorts of information.

WO 2005/102 144 A1 discloses an endoscope having input means enabling a user to input various sorts of information.

### Disclosure of Invention

### Means for Solving the Problem

To achieve the above-described object, the present invention provides an endoscopic system according to claim 1 including an endoscope having an input portion, a monitor which displays an image taken with the endoscope, an observation apparatus which is detachably attached to the endoscope, and which, when connected to the endoscope, automatically displays on the monitor a setting view on which a setting can be made through the input portion.

### Brief Description of the Drawings

Fig. 1 is an entire configuration diagram showing an endoscopic system according to a first embodiment of the present invention;
Fig. 2 is a plan view showing a monitor screen activated when an electronic endoscope and an observation apparatus are connected to each other according to the first embodiment;
Fig. 3 is a plan view showing the monitor screen when patient information is inputted in Japanese (by kana-kanji conversion) according to the first embodiment;
Fig. 4 is a plan view showing the monitor screen when patient information is input in alphabetic character form according to the first embodiment;
Fig. 5 is a plan view showing the monitor screen when patient information is inputted in numeric form according to the first embodiment;
Fig. 6 is a plan view showing the monitor screen when a confirmation view is displayed after input of patient information according to the first embodiment;
Fig. 7 is a plan view showing a first modified example and showing the monitor screen when patient information is inputted in Japanese (by kana-kanji conversion) according to the first embodiment;
Fig. 8 is a plan view showing a second modified example and showing the monitor screen when patient information is inputted in Japanese (by kana-kanji conversion) by means of a touch panel system, while an endoscopic image is being displayed in the background according to the first embodiment;
Fig. 9 is a plan view showing a third modified example and showing a configuration of a software keyboard according to the first embodiment;
Fig. 10 is an entire configuration diagram showing an endoscopic system according to a second embodiment of the present invention; and
Fig. 11 is a plan view showing a monitor screen activated when an electronic endoscope and an observation apparatus are connected to each other, and when an insertion portion and an operation portion of the electronic endoscope are connected to each other according to the second embodiment.

### Best Mode for Carrying Out the Invention

Embodiments of the present invention will be described below. With respect to each of endoscopic systems according to the embodiments, character lines in the language notation used mainly in Japan will be described by way of example in the following description.

### (First Embodiment)

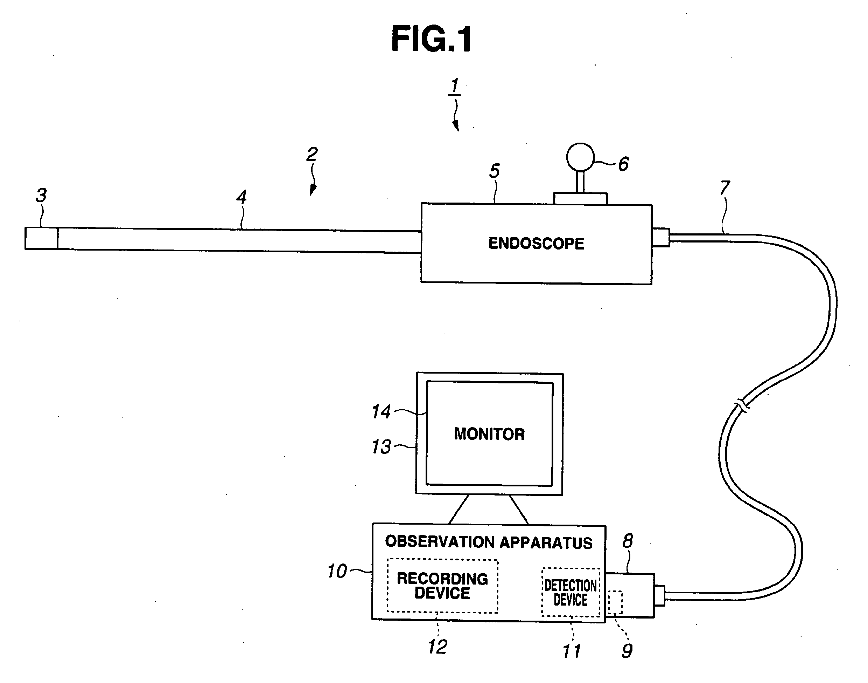

A first embodiment of the present invention will be described with reference to Figs. 1 to 9.

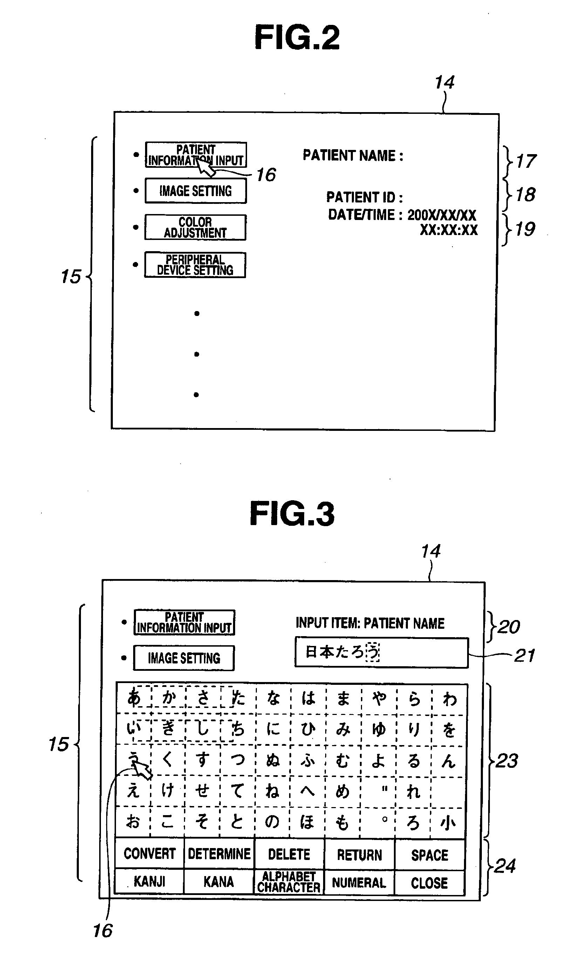

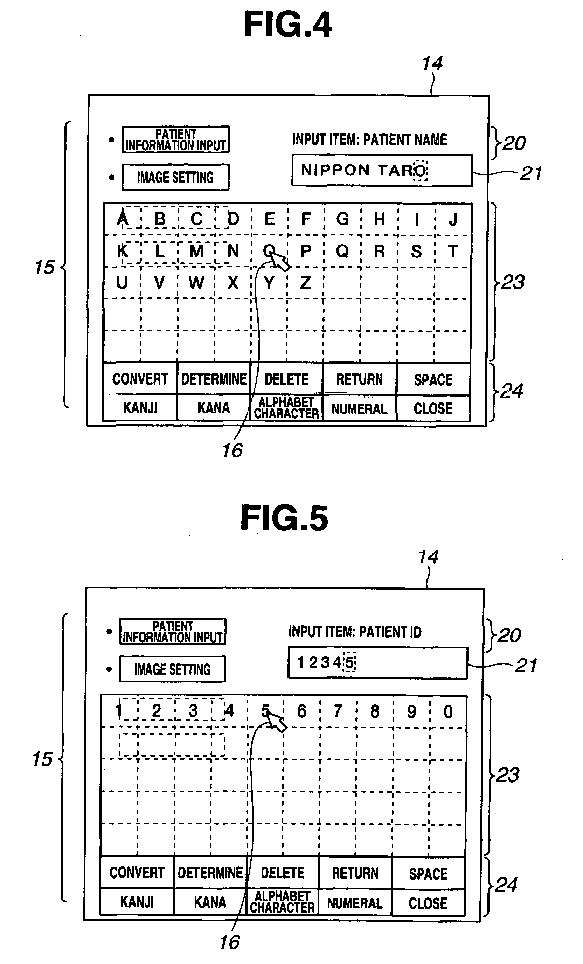

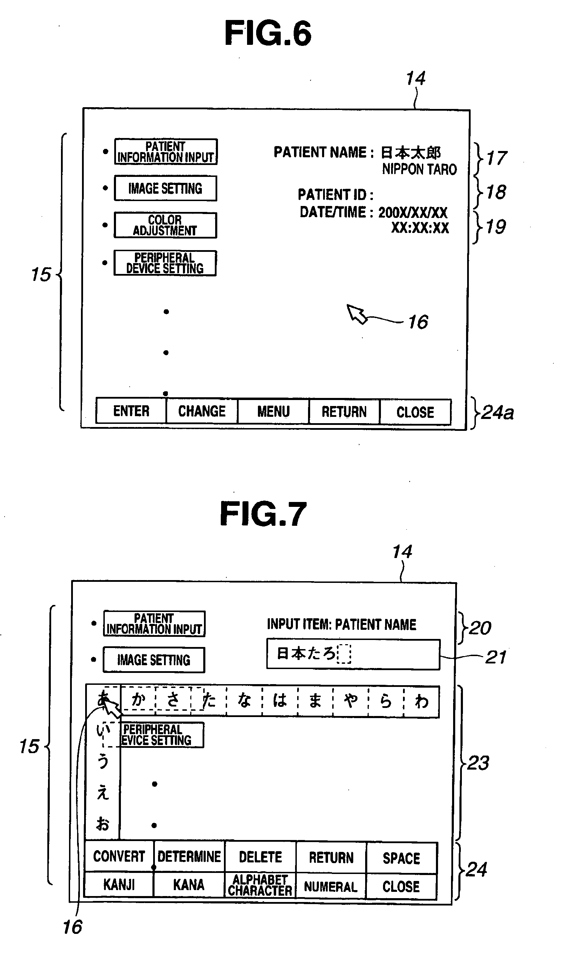

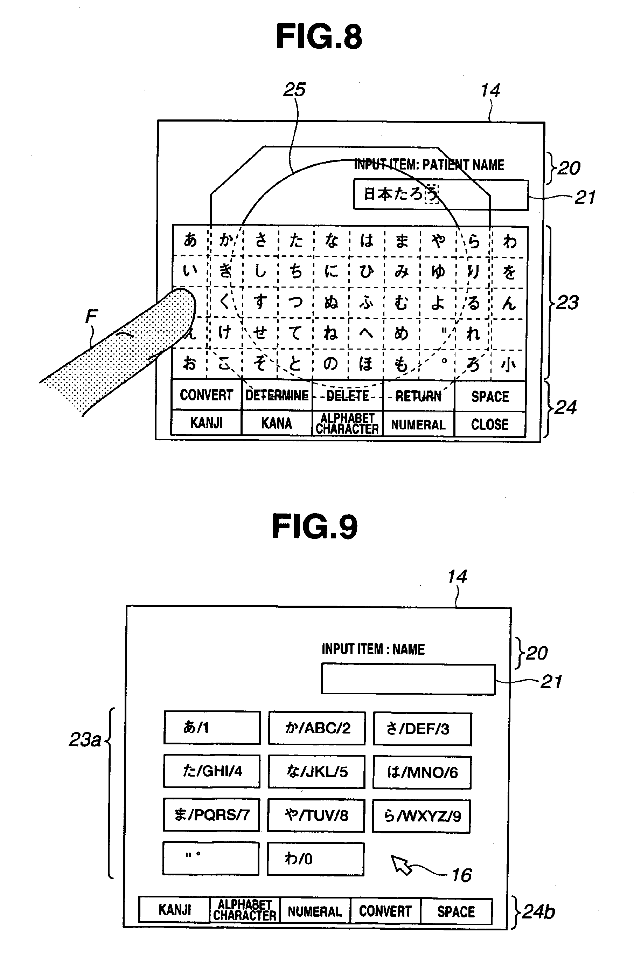

Figs. 1 to 9 relates to a description of a first embodiment of an endoscopic system according to the present invention. Fig. 1 is an entire configuration diagram showing the endoscopic system. Fig. 2 is a plan view showing a monitor screen activated when an electronic endoscope and an observation apparatus are connected to each other. Fig. 3 is a plan view showing the monitor screen when patient information is inputted in Japanese (by kana-kanji conversion). Fig. 4 is a plan view showing the monitor screen when patient information is input in alphabetic character form. Fig. 5 is a plan view showing the monitor screen when patient information is inputted in numeric form. Fig. 6 is a plan view showing the monitor screen when a confirmation view is displayed after input of patient information. Fig. 7 is a plan view showing a first modified example and showing the monitor screen when patient information is inputted in Japanese (by kana-kanji conversion). Fig. 8 is a plan view showing a second modified example and showing the monitor screen when patient information is inputted in Japanese (by kana-kanji conversion) by means of a touch panel system, while an endoscopic image is being displayed in the background. Fig. 9 is a plan view showing a third modified example and showing a configuration of a software keyboard.

As shown in Fig. 1, an endoscopic system 1 in the present embodiment is configured of an electronic endoscope 2 (hereinafter referred to simply as endoscope), an observation apparatus 10, which is a camera control unit (CCU), and a monitor 13, which is a display device.

The endoscope 2 is configured of in order from the distal end, a distal end portion 3, an insertion portion 4 formed continuously with the distal end portion, an operation portion 5 connected to a proximal end of the insertion portion 4, and a universal cord 7, which is an electric cable extending from the operation portion 5.

The distal end portion 3 of the endoscope 2 is provided with an image pickup unit not shown in the figure. The image pickup unit incorporates illumination means and image pickup means in the present embodiment. The illumination means of the image pickup unit is configured, for example, as an LED serving as a light source in itself or configured to radiate light by guiding illumination light from an external light source through a light guide fiber passed through the insertion portion 4, the operation portion 5 and the universal cord 7. As the image pickup means in the image pickup unit, an image pickup device, e.g., a CCD or CMOS is used. The image pickup means is configured to take an endoscopic image through the distal end portion 3 or an image guide fiber.

The insertion portion 4 is a flexible endoscope insertion portion or a rigid endoscope insertion portion. If the insertion portion 4 is the flexible endoscope insertion portion, it may have a bending portion in its distal end portion.

The operation portion 5 is provided with an operating lever 6, which is a joystick-type input portion provided as a pointing device. The operating lever 6 is not limited to a joystick-type pointing device. For example, the operating lever 6 may be a track ball.

The universal cord 7 has a connection portion 8 at its extended end. The connection portion 8 is detachably connected electrically to the observation apparatus 10. A detection chip 9, which is a component part to be detected, is incorporated in the connection portion 8.

The observation apparatus 10 incorporates a detection device 11, which is a detection portion for detecting the detection chip 9 when the connection portion 8 is electrically connected, a recording device 12, which records endoscopic images taken with the above-described electronic endoscope 2, and a processor (not shown).

The detection device 11 simply recognizes having detected the connection portion 8 of the universal cord 7. The detection device 11 may be a device for electrically detecting the above-described detection chip 9 or a mechanical connection detection switch.

The recording device 12 in the present embodiment is a device capable of writing the above-mentioned endoscopic images on recording medium, e.g., a hard disk, any of various types of disks, a flash memory, or any of various types of picture cards. The recording device 12 may be externally mounted instead of being limited to the one incorporated in the observation apparatus 10.

The processor (not shown) performs image processing on a photoelectrically converted image signal from the endoscope 2 to enable the signal to be displayed as an endoscopic image on a monitor 13. A detection signal from the detection device 11 is inputted to the processor.

The monitor 13 is connected to the observation apparatus 10 and displays endoscopic images and various setting views on a monitor screen 14.

The operation of the thus-configured endoscopic system 1 in the present embodiment will next be described with reference to Figs. 2 to 6.

A user, who is an operator, first prepares the endoscopic system 1 for endoscopic inspection. At this time, the user connects the endoscope 2 and the observation apparatus 10 to each other. That is, the user couples and connects the connection portion 8 of the universal cord 7 of the endoscope 2 to the observation apparatus 10 at a predetermined connection position on the observation apparatus 10.

When the endoscope 2 and the observation apparatus 10 are connected to each other, the detection device 11 in the observation apparatus 10 detects the detection chip 9 in the connection portion 8 and outputs the detection signal to the processor. An initial setting view such as shown in Fig. 2 is then displayed (activated) on the monitor screen 14.

In the initial setting view displayed on the monitor screen 14, a group of setting tabs 15, which are command buttons including a plurality of setting items, such as a patient information input tab, an image setting tab, a color adjustment tab and a peripheral device setting tab, a patient name display portion 17, a patient ID display portion 18, a date/time display portion 19, the date of birth of a patient not shown in the figure, etc., are displayed. In the present embodiment, the group of setting tabs 15 are displayed at the left-hand side of the monitor screen 14, while the display portions 17 to 19 are displayed at the right-hand side of the monitor screen 14.

On the monitor screen 14, a selecting pointer 16 freely operable with the operating lever 6 provided on the operation portion 5 of the endoscope 2 is also displayed. The selecting pointer 16 is moved in the monitor screen 14 by being linked to the operation of the operating lever 6.

The user moves the selecting pointer 16, for example, onto the patient setting input in the group of setting tabs 15, as shown in Fig. 2, and depresses the operating lever 6 to change the display to a patient information setting view. An input item display portion 20 and an input cell 21 are then displayed in an upper right portion of the monitor screen 14, and a software keyboard 23 having hiragana character lines and a group of change keys 24 including a plurality of change keys are displayed (activated) from a central portion to a lower portion. At this time, the software keyboard 23 and the group of change keys 24 are displayed in a transparent display manner as shown in Fig. 3, such that the group of setting tabs 15 in the menu view shown in Fig. 2 are displayed in the background.

The user moves the selecting pointer 16 onto one of the hiragana characters that he or she wants to select on the hiragana character software keyboard 23 formed of an array of the kana syllabary by operating the operating lever 6 on the operation portion 5 when inputting a patient name in Japanese, and depresses (clicks) the operating lever 6. The selected hiragana character is then displayed in the input cell 21, as shown in Fig. 3.

Also, if the user wants to convert the hiragana character into a kanji or a katakana character, he or she places on the kanji key or the kana key in the group of change keys 24 and depresses (clicks) the operating lever 6. If the user wants to convert the selected hiragana character into a small letter, he or she can convert the selected character by again (double) clicking the selected character or by clicking the small letter on the software keyboard 23. If the user wants to input a space, he or she can do so by selecting a space key in the group of change keys 24. Further, conversion candidates for the converted kanji are successively displayed by clicking the kanji key in the group of change keys 24.

In the endoscopic system 1 in the present embodiment, alphabet (English character) input and numeral input can also be performed by selecting an alphabetic character key or a numeral key in the group of change keys 24.

When the user clicks the alphabetic character key in the group of change keys 24, the software keyboard 23 is changed for another having alphabetic character lines, as shown in Fig. 4. The user can then input a patient name in the alphabet by moving the selecting pointer 16 to each of the alphabetic characters that he or she wants to select and by depressing (clicking) the operating lever 6, as he or she does in the case of inputting hiragana characters.

If the user wants to convert the selected alphabetic character into a small letter, he or she can convert the selected character by again (double) clicking the selected character.

Also, when the user clicks the numeral key in the group of change keys 24, the software keyboard 23 is changed for another having numeric character lines, as shown in Fig. 5. Also in this case, the user can input any of various IDs in numeric form by moving the selecting pointer 16 to each of the numeric characters that he or she wants to select and by depressing (clicking) the operating lever 6. The above-mentioned various IDs include a patient ID, a doctor ID, an endoscope ID and a management ID of the recording medium on which a recording is made. The character information inputted by means of the software keyboard 23 is written to the recording medium in the recording device 12 in the observation apparatus 10 along with the endoscopic image.

In the present embodiment, the input items are changed in order of a patient name and a patient ID. When the user selects a determination key in the group of change keys 24 after inputting characters for the corresponding information, a change to a next input item is made. The user can also make a return to the immediately preceding input item by selecting a return key in the group of change keys 24.

When the user can switch off the input item display portion 20, the input cell 21 and the software keyboard 23 on the monitor screen 14 by selecting a close key in the group of change keys 24 after inputting various input items, as shown in Fig. 6.

At this time, the display on the monitor screen 14 is returned to the initial view in which the group of setting tabs 15 are displayed, and the information inputted at the right-hand side is displayed in the display portions 17 to 19. Also, a group of change keys 24a including an enter key, a change key, a menu key, a return key and a close key are displayed in a lowermost portion of the monitor screen 14. While in the present embodiment only a patient name, a patient ID and a date are shown in Fig. 6, various sorts of information such as a doctor ID and an endoscope ID may also be displayed.

If the user wants to switch from this initial view to an endoscopic image, he or she can do so by selecting the close key in the group of change keys 24a. Also, if the user wants to change some of the various sorts of information inputted, he or she can select the change key in the group of change keys 24a or again select the patient information input tab in the group of setting tabs 15 to change the information by the above-described procedure.

As described above, the endoscopic system 1 in the present embodiment enables the above-described various sorts of information to be inputted by means of the operating lever 6 provided on the operation portion 5 of the endoscope 2, by selecting from the group of setting tabs 15 displayed on the monitor screen 14 and by inputting from the software keyboard 23, even though the observation apparatus 10 is provided with no such input device as a keyboard. Thus, the endoscopic system 1 can be configured in a compact form without requiring any additional input device.

Thus, the endoscopic system 1 in the present embodiment does not reduce the in-hospital space in which treatments are made and does not place any input device for inputting various sorts of information as an obstacle in a manipulation action region close to a patient. As a result, a user who is a doctor can easily perform manipulations.

Also, since the endoscopic system 1 in the present embodiment does not require any input device, a sterilization treatment may be performed only on the endoscope 2 after the operation. Therefore, sterilized endoscopes 2 may be simply connected at the time of continuous endoscopic inspection, thus improving the availability factor of the endoscopic system 1.

The software keyboard 23 may be configured to have only the characters at the heads of the a- to wa-rows displayed, as shown in Fig. 7, and to enable selection from the characters by displaying, for example, the characters in the a-row (a to o) when the selecting pointer 16 is placed on the a-row head character (a). The software keyboard 23 thus configured is capable of increasing the display area in the background view.

The software keyboard 23 may alternatively be of a touch panel type, such that selection can be made with finger F, as shown in Fig. 8. Also, the input item display portion 20, the input cell 21, the software keyboard 23 and the group of change keys 24 may be configured to enable display of the background view on an endoscopic image 25 as well, as shown in Fig. 8, as well as to enable input of various sorts of information.

Further, a software keyboard 23a may be configured to enable characters in one of various character lines to be selected one after another each time the corresponding character line key is selected by being clicked, as shown in Fig. 9. More specifically, any one of the characters in each of the a- to wa-rows can be selected according to the number of times the corresponding key is clicked. Input of alphabetic or numeric characters is enabled by selecting an alphabetic character or a numeric character in a group of change keys 24b, and selection can be made according to the number of times the displayed character is clicked.

While the above-described endoscopic system 1 in the present embodiment is configured so that selection from operations is freely made on the monitor screen 14 by means of the selecting pointer 16 or a touch panel, a configuration, e.g., one including a cursor for indicating a selected position in a highlighting manner may alternatively be adopted.

### (Second Embodiment)

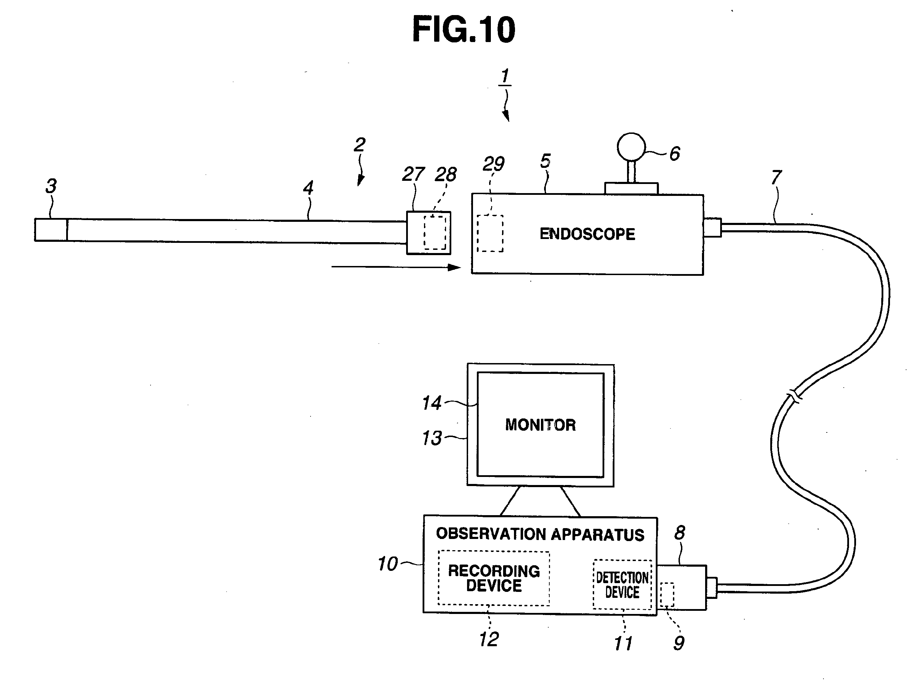

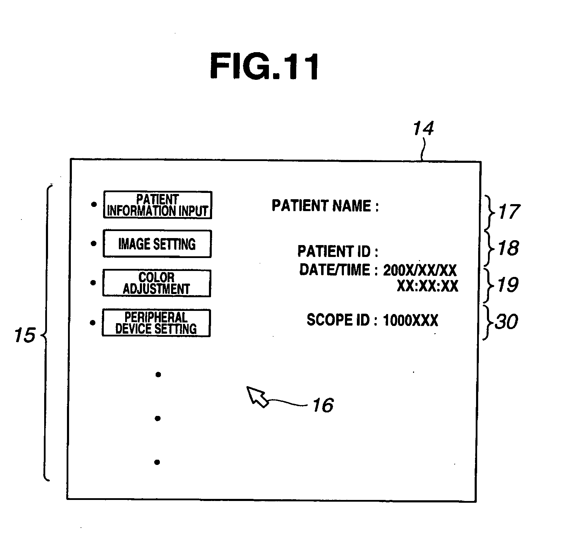

A second embodiment of the present invention will next be described with reference to Figs. 10 and 11.

Figs. 10 and 11 relates to a description of a second embodiment of the endoscopic system according to the present invention. Fig. 10 is an entire configuration diagram showing the endoscopic system. Fig. 11 is a plan view showing a monitor screen activated when an electronic endoscope and an observation apparatus are connected to each other, and when an insertion portion and an operation portion of the electronic endoscope are connected to each other.

An endoscope 2 in an endoscopic system 1 in the present embodiment is configured so that an insertion portion 4 and an operation portion 5 are detachably connected to each other. In the following description, the same reference numerals are used for the same configuration as that of the above-described endoscopic system 1 in the first embodiment, and the detailed description of the same configuration will not be repeated.

The insertion portion 4 of the endoscope 2 has an insertion portion connector 27 on its proximal end portion. The insertion portion connector 27 incorporates an ID chip 28 in which an endoscope ID is recorded and is connected to the operation portion 5. The operation portion 5 incorporates a detection portion 29 with which the ID chip 28 in the insertion portion connector 27 is read. The detection portion 29 is electrically connected to an observation apparatus 10 via a universal cord 7.

The insertion portion 4 in the present embodiment is of a one-time-use (disposable) type, is used for each patient and is subjected to medical disposal after being used. Needless to say, the insertion portion 4 may alternatively be of a reusable type.

In the endoscopic system 1 in the present embodiment, the connection portion 8 of the universal cord 7 of the endoscope 2 is connected to the observation apparatus 10 before endoscopic inspection, as is that in the first embodiment. The insertion portion connector 27 of the insertion portion 2 is also connected to the operation portion 29.

At this time, the detection portion 29 in the operation portion 5 detects the ID chip 28 in the insertion portion connector 27 and outputs information on the completion of connection between the insertion portion 4 and the operation portion 5 and endoscope ID information stored in the ID chip 28 to the observation apparatus 10.

An initial setting view such as shown in Fig. 11 is then displayed (activated) on the monitor screen 14, as is that in the first embodiment. An endoscope ID number, which is identification information, is automatically displayed in an endoscope ID (SCOPE ID) display portion 30 below the date/time display portion 19 of the monitor screen 14.

If the insertion portion 4 of the endoscope 2 is disposable, identification information for identifying a state after use is written to the ID chip 28 from the detection portion 29 when the insertion portion 4 is connected to the operation portion 5. This configuration enables the detection portion 29 to recognize the insertion portion 4 of the endoscope 2 used once as having been used from the information in the ID chip 28.

The detection portion 29 detecting the information indicating that the insertion portion 4 has been used outputs a detection signal to the observation apparatus 10. The observation apparatus 10 then produces on the monitor screen 14 a warning display indicating that the insertion portion 4 has been used.

The endoscopic system 1 in the present embodiment is configured as described above to save work for inputting the endoscope ID and to prevent erroneous use of the used disposable-type insertion portion 4. Other operations including an operation to input patient information are the same as those in the first embodiment.

The endoscopic system in each embodiment of the present invention is compact, not existing as an obstacle in a user manipulation action region, and makes it possible to easily input various sorts of information.

With respect to the present embodiment, the language notation of the software keyboards 23 and 23a for performing operations displayed on the monitor screen 14 in the embodiments has been described by mainly using character lines used in Japan. However, needless to say, analogous use of the software keyboard 23 or 23 a on the monitor screen 14 may be made so that character lines in a language notation in any country where the endoscopic system 1 is to be used can be inputted by operating the software keyboard 23 or 23a.

The above-described invention is not limited to the embodiments. Various modifications can be made without departing from the gist of the invention within the scope of the claims in an implementation stage. Also, the embodiments include different forms of the invention in various stages, and various forms of the invention can be extracted according to suitable combinations of the plurality of constituent features disclosed.

For example, even in a case where several ones of all the constituent features described with respect to the embodiments are removed, the configuration from which these constituent features are removed can be extracted as the invention if the problems described in the section "Problems to be Solved by the Invention" can be solved, and if the advantages described in "Advantages of the invention" can be obtained.

## Claims

1. An endoscopic system (1) comprising:
an endoscope(2) having an input portion (6) and a connection portion (8);
a monitor (13) configured to display an image taken with the endoscope (2);
an observation apparatus (10) to which the connection portion (8) of the endoscope (2) is detachably attached, and which includes a detection device (11) and is connected to the monitor (13),
**characterized in that**
the connection portion (8) incorporates a detection chip (9), and
the observation apparatus (10) is configured to detect the detection chip (9) of the connection portion (8) and automatically display on the monitor (13) a setting view on which a setting can be made through the input portion (6) when the connection portion (8) of the endoscope (2) is connected to the observation apparatus (10).

2. The endoscopic system (1) according to claim 1, wherein command buttons (15) for inputting various sorts of information are displayed on the operation view from the input portion (6).

3. The endoscopic system (1) according to claim 2, wherein the observation apparatus (10) is configured to display a software keyboard (23) on the monitor (13) through which characters can be input on a screen by the input portion (6) according to a selection from the command buttons (15).

4. The endoscopic system (1) according to any one of claims 1 to 3, wherein the endoscope (2) includes: an operation portion (5); and an insertion portion (4) which is configured to be detachably attached to the operation portion (5), and in which identification information is recorded, and
wherein when the operation portion (5) and the insertion portion (4) are connected to each other, the identification information is outputted to the observation apparatus (10) to be automatically displayed in the setting view.

5. The endoscopic system (1) according to claim 3, wherein the observation apparatus (10) has a recording device (12), and
the recording device (12) is configured to record character information inputted through the software keyboard (23) along with the image taken with the endoscope (2).

6. The endoscopic system (1) according to claim 4, wherein the observation apparatus (10) has a recording device (12), and
the recording device (12) is configured to record the identification information along with the endoscopic image taken with the endoscope (2).

7. The endoscopic system (1) according to any one of claims 1 to 6, wherein the input portion (6) is a pointing device.

8. The endoscopic system (1) according to claims 3, 5 or 6, wherein the software keyboard (23) is a touch panel which can be set on the screen in addition to the input portion (6).

9. The endoscopic system (1) according to any one of claims 1 to 8, wherein the monitor (13) is configured to display the setting view on an endoscopic image, with the endoscopic image serving as a background view.

## Patentansprüche

1. Endoskopsystem (1) aufweisend:
ein Endoskop (2) mit einem Eingabeteil (6) und einem Verbindungsteil (8);
einen Monitor (13), der dazu eingerichtet ist, ein mit dem Endoskop (2) aufgenommenes Bild anzuzeigen;
eine Beobachtungsvorrichtung (10), mit dem der Verbindungsteil (8) des Endoskops (2) lösbar verbunden ist, und die eine Erfassungsvorrichtung (11) enthält und mit dem Monitor (13) verbunden ist,
**dadurch gekennzeichnet, dass**
der Verbindungsteil (8) einen Erfassungschip (9) beinhaltet, und
die Beobachtungsvorrichtung (10) dazu eingerichtet ist, den Erfassungschip (9) des Verbindungsteils (8) zu erfassen und auf dem Monitor (13) automatisch eine Einstellungsansicht anzuzeigen, in der eine Einstellung durch den Eingabeteil (6) gemacht werden kann, wenn der Verbindungsteil (8) des Endoskops (2) mit der Beobachtungsvorrichtung (10) verbunden ist.

2. Endoskopsystem (1) nach Anspruch 1, bei dem Befehlsknöpfe (15) zum Eingaben verschiedener Arten von Information durch den Eingabeteil (6) in der Betätigungsansicht angezeigt werden.

3. Endoskopsystem (1) nach Anspruch 1, bei dem die Beobachtungsvorrichtung (10) dazu eingerichtet ist, in dem Monitor (13) eine Softwaretastatur (23) anzuzeigen, durch welche durch den Eingabeteil (6) gemäß einer Auswahl von den Befehlsknöpfen (15) Zeichen auf einem Bildschirm eingegeben werden können.

4. Endoskopsystm (1) nach einem der Ansprüche 1 bis 3, bei dem das Endoskop (2) enthält: einen Befehlsteil (5); und einen Einführungsteil (4), der dazu eingerichtet ist, lösbar an dem Betätigungsteil (5) angebracht zu werden, und in dem die Identifikationsinformation aufgenommen wird, und
wobei, wenn der Betätigungsteil (5) und der Einführungsteil (4) miteinander verbunden sind, wird die Identifikationsinformation zu der Beobachtungsvorrichtung (10) ausgegeben, um automatisch in der Einstellungsansicht angezeigt zu werden.

5. Endoskopsystem (1) nach Anspruch 3, bei dem die Beobachtungsvorrichtung (10) eine Aufnahmevorrichtung (12) aufweist, und
die Aufnahmevorrichtung (12) dazu eingerichtet ist, durch die Softwaretastatur (23) eingegebene Zeicheninformation zusammen mit dem durch das Endoskop (2) aufgenommene Bild aufzunehmen.

6. Endoskopsystem (1) nach Anspruch 4, bei dem die Beobachtungsvorrichtung (10) eine Aufnahmevorrichtung (12) aufweist, und
die Aufnahmevorrichtung (12) dazu eingerichtet ist, die Identifikationsinformation zusammen mit dem durch das Endoskop (2) aufgenommenen endoskopischen Bild aufzunehmen.

7. Endoskopsystem (1) nach einem der Ansprüche 1 bis 6, bei dem der Eingabeteil (6) ein Zeigegerät ist.

8. Endoskopsystem (1) nach den Ansprüchen 3, 5 oder 6, bei dem die Softwaretastatur (23) ein Bildschirm-Tastenfeld ist, das zusätzlich zu dem Eingabeteil (6) auf den Bildschirm platziert werden kann.

9. Endoskopsystem (1) nach einem der Ansprüche 1 bis 8, bei dem der Monitor (13) dazu eingerichtet ist, die Einstellungsansicht auf einem endoskopischen Bild anzuzeigen, wobei das endoskopische Bild als eine Hintergrundansicht dient.

## Revendications

1. Système (1) endoscopique comprenant :
un endoscope (2) comportant une partie (6) d'entrée et une partie (8) de connexion ;
un moniteur (13) configuré pour afficher une image prise avec l'endoscope (2) ;
un appareil (10) d'observation auquel la partie (8) de connexion de l'endoscope (2) est fixée de manière détachable, et qui comprend un dispositif (11) de détection et est connecté au moniteur (13),
**caractérisé en ce que**
la partie (8) de connexion incorpore une puce (9) de détection, et
l'appareil (10) d'observation est configuré pour détecter la puce (9) de détection de la partie (8) de connexion et afficher automatiquement sur le moniteur (13) une vue de réglage sur laquelle un réglage peut être effectué par l'intermédiaire de la partie (6) d'entrée lorsque la partie (8) de connexion de l'endoscope (2) est connectée à l'appareil (10) d'observation.

2. Système (1) endoscopique selon la revendication 1, dans lequel des boutons (15) de commande destinés à délivrer en entrées diverses sortes d'informations sont affichés sur la vue opérationnelle à partir de la partie (6) d'entrée.

3. Système (1) endoscopique selon la revendication 2, dans lequel l'appareil (10) d'observation est configuré pour afficher un clavier (23) logiciel sur le moniteur (13) par l'intermédiaire duquel des caractères peuvent être délivrés en entrée sur un écran par la partie (6) d'entrée conformément à une sélection à partir des boutons (15) de commande.

4. Système (1) endoscopique selon l'une quelconque des revendications 1 à 3, dans lequel l'endoscope (2) comprend : une partie (5) d'actionnement ; et une partie (4) d'insertion qui est configurée pour être fixée de manière détachable à la partie (5) d'actionnement, et dans laquelle des informations d'identification sont enregistrées, et
dans lequel lorsque la partie (5) d'actionnement et la partie (4) d'insertion sont connectées l'une à l'autre, les informations d'identification sont délivrées en sortie vers l'appareil (10) d'observation pour être automatiquement affichées dans la vue de réglage.

5. Système (1) endoscopique selon la revendication 3, dans lequel l'appareil (10) d'observation comporte un dispositif (12) d'enregistrement, et
le dispositif (12) d'enregistrement est configuré pour enregistrer des informations de caractères délivrées en entrée par l'intermédiaire du clavier (23) logiciel avec l'image prise avec l'endoscope (2).

6. Système (1) endoscopique selon la revendication 4, dans lequel l'appareil (10) d'observation comporte un dispositif (12) d'enregistrement, et
le dispositif (12) d'enregistrement est configuré pour enregistrer les informations d'identification avec l'image endoscopique prise avec l'endoscope (2).

7. Système (1) endoscopique selon l'une quelconque des revendications 1 à 6, dans lequel la partie (6) d'entrée est un dispositif de pointage.

8. Système (1) endoscopique selon les revendications 3, 5 ou 6, dans lequel le clavier (23) logiciel est un panneau tactile qui peut être établi sur l'écran en plus de la partie (6) d'entrée.

9. Système (1) endoscopique selon l'une quelconque des revendications 1 à 8, dans lequel le moniteur (13) est configuré pour afficher la vue de réglage sur une image endoscopique, l'image endoscopique servant de vue d'arrière-plan.
